# EUROPEAN PATENT SPECIFICATION

(11) **EP 3 069 678 B1**
(45) Date of publication and mention of the grant of the patent: **24.01.2018**
(21) Application number: 16161272.6
(22) Date of filing: 18.03.2016
(51) Int. Cl.: A61B 18/14, A61C 1/07, A61N 7/00

(54) **HANDHELD RF OR ULTRASONIC DEVICE**
HOCHFREQUENZ- ODER ULTRASCHALL-HANDGERÄT
APPAREIL PORTATIF À HAUTE FRÉQUENCE OU À ULTRASONS

(30) Priority: 18.03.2015 IT MI20150410
(43) Date of publication of application: 21.09.2016
(73) Proprietor: MDM Group S.r.l., 40010 Sala Bolognese (BO) (IT)
(72) Inventor: Montanari, Federico, 40010 Sala Bolognese BO (IT)
(74) Representative: Perani & Partners S.p.A.

(56) References cited:
- US-A- 5 501 596
- US-A1- 2011 130 664
- US-A1- 2014 031 726

## Description

### FIELD OF APPLICATION

The present description relates to a handpiece for emitting radiofrequency or ultrasound waves, particularly applicable to radio-frequency or ultrasound treatments, according to the preamble of claim 1.

### Description of the prior art

Nowadays, mitigating the effects of aging and the damage to the skin from sun exposure is strongly felt, as these are the major root causes of superficial and deep wrinkles on the face.

It is known in the art to make a handpiece capable of emitting radio-frequency or ultrasound waves to perform radio-frequency or ultrasound treatments, respectively.

In particular, such handpieces have a tip configured to emit radio-frequency or ultrasound waves when placed in contact with an individual's skin.

These handpieces may be of the monopolar or bipolar type and, in the case of bipolar, these appear to be more indicated for the treatment of the individual's skin.

The structure of the bipolar handpiece encloses an actuator and has, in a limited zone, a neutral electrode and an active electrode (either resistive or capacitive). In this way, the current tends to circulate in the underlying tissue by migrating from one electrode to the other, affecting the average deep tissues.

### Drawback of the prior art

For applications where high accuracy is required, such as for the selective treatment of wrinkles, handpieces are used which use a needle to treat very restricted portions of the dermis by radio-frequency or ultrasounds. However, these handpieces have the drawback of being highly invasive since the needle must necessarily penetrate the skin in order to radiate the desired zone with the radio-frequency or ultrasounds.

Furthermore, "invasive" handpieces using a needle are of the disposable type. While "non-invasive" handpieces using an actuator having a tip intended to come into contact with the skin surface without damaging it still have the drawback of not being able to be sterilized due the presence of electrical components inside them.

Reference is made to documents US5501596 and US2011/0130664 A1.

### SUMMARY OF THE INVENTION

The object of the present invention is to provide a handpiece for radio-frequency or ultrasounds which is versatile and adaptable to a multiplicity of applications.

A further object of the present invention is to provide a sterilizable handpiece, in particular autoclavable.

The invention is defined in the appended claims.

### Advantages of the invention

With one embodiment, it is possible to implement a sterilizable and reusable handpiece.

With one embodiment, it is possible to implement a highly versatile handpiece adaptable to different types of applications, usable in treatments of zones such as wrinkles around the lips and the so-called "bar code", periocular wrinkles and dark circles-bags zone, ptosis of the fixed eyelid and forehead and glabellar wrinkles or in any case, in applications where the use of a sterile handpiece is required.

With one embodiment, it is possible to implement a handpiece suitable for intraoral dental applications.

### BRIEF DESCRIPTION OF THE DRAWINGS

The features and the advantages of the present invention will appear more clearly from the following detailed description of a possible practical embodiment thereof, shown by way of a non-limiting example in the set of drawings, in which:
- figure 1 shows a handpiece of the present invention,
- figure 2 shows a view of a detail of the handpiece of the present invention,
- figure 3 shows a view of another detail of the handpiece in figure 1,
- figures 4 and 5 show a sectional view of the detail in figure 2, in the absence (Figure 4) or in the presence (Figure 5) of insulating resin,
- figure 6 shows a sectional view of the detail in figure 3.

### DETAILED DESCRIPTION

The handpiece shown in the accompanying figures shall be considered to be represented schematically, not necessarily in scale and not necessarily with the proportions shown between the various component elements.

Although not explicitly mentioned, the single features described with reference to the specific embodiments shall be considered as ancillary and/or interchangeable with other features, which are described with reference to other exemplary embodiments.

Hereinafter, for brevity of description, reference will be made without limitation to insulating means, such as materials having fluid-impermeability and/or thermal and electric insulation properties.

In particular, insulating materials are all the materials able to withstand rapid changes in pressure, temperature and liquids.

Hereinafter, for brevity of description, reference will be made without limitation to the term autoclavable as the ability of an object to be sterilized in an autoclave.

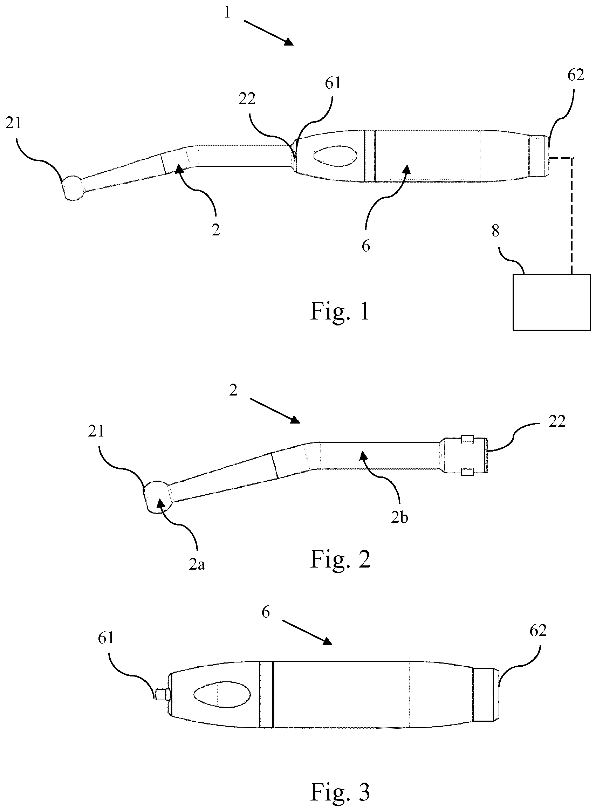

The object of the present invention is a handpiece 1 (see figures 1-3) for emitting electromagnetic radio-frequency or ultrasound waves.

Handpiece 1 comprises a body 2 and a handle 6 which can be coupled to each other to create a mechanical and electrical coupling. Such a coupling is of the reversible type, i.e. it allows mechanically and electrically coupling or uncoupling body 2 from handle 6 when body 2 must be replaced for cleaning operations or to use a body having different specifications.

In particular, body 2 extends along a predefined extension direction between a first head end 21 and a second end 22.

Such a second end 22 is distal with respect to the first head end and represents the tail of body 2.

Body 2 comprises a resonant actuator 3 configured to generate electromagnetic radio-frequency or ultrasound waves and it comprises electrical connection means 4 configured to power the resonant actuator 3.

Body 2 is shaped to accommodate the resonant actuator 3 close to the first head end 21 and to accommodate the electrical connection means 4 close to the second end 22.

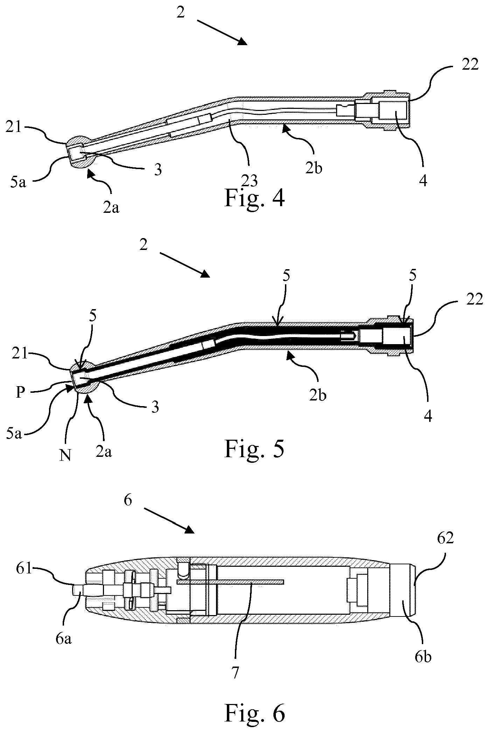

To this end, body 2 is hollow and defines an inner cavity 23 extending between the first head end 21 and the second end 22 (see figures 4 and 5).

The inner cavity 23 is therefore suitable for accommodating the resonant actuator 3 close to the first head end 21 and the electrical connection means 4 close to the second end 22.

Advantageously, handpiece 1 comprises insulating means 5 arranged in cavity 23 to make body 2 sterilizable in autoclave.

For example, the insulating means 5 are arranged close to the first head end 21 and to the second end 22 to make body 2 sterilizable in autoclave or, in a preferred manner, the insulating means 5 are spread into cavity 23 between the first head end 21 and the second end 22 so as to enclose or incorporate the resonant actuator 3 and the electrical connection means 4.

Preferably, the insulating means 5 consist of an insulating polymer material such as, for example, an insulating polymer resin.

The use of the polymer resin allows obtaining a high degree of insulation and consequently of resistance to extreme corrosive environments.

Even more preferably, the insulating polymer resin is a two-component epoxy resin.

In particular, in order to deliver the radiofrequency or ultrasound waves to the body surface portion, the first head end 21 of body 2 comprises an internally hollow tip 2a.

Tip 2a defines such a volume as to accommodate the resonant actuator 3.

Body 2 further comprises a neck 2b, for example shaped as a hollow cylindrical element extending between the second end 22 and the first head end 21, so as to couple with tip 2a.

In other words, the inner cavity 23 extends with continuity passing inside neck 2b and tip 2a and preferably has a substantially cylindrical section.

Neck 2b is configured to accommodate the electrical connection means 4.

According to one aspect, the insulating means 5 are placed within the inner cavity 23 close to tip 2a and to neck 2b close to said second end 22.

According to a preferred solution, handpiece 1 is a bipolar handpiece.

To this end, tip 2a is made of a biocompatible material in which a part is composed of conductive material, so as to form an "exposed" negative pole N, and a part is provided with suitable electrical insulation means 5a, which form an "insulated" positive pole P.

In particular, the "Exposed Negative Pole " N is made of a biocompatible material (e.g. stainless steel 313), while the "Insulated Positive Pole" P is made of a biocompatible high-voltage insulated material (e.g. polyamide, polytetrafluoroethylene).

According to one aspect, the "Exposed Negative Pole" N is localized on the outer surface of tip 2a, while the "Insulated Positive Pole" P is localized in the central and inner part of tip 2a.

It should be noted that handpiece 1 is configured to emit the radio-frequency waves when the "Insulated Positive Pole" P and the "Exposed Negative Pole" N are simultaneously in contact with a patient's body surface portion.

With reference to Figures 1 and 2, it is noted that tip 2a of body 2 is shaped as a sphere in such a way as to enable a good contact with an individual's body surface portion.

To this end, the sphere of tip 2a has an outer diameter of between 10mm and 16mm.

According to a preferred embodiment, neck 2b is shaped as an elongated hollow cylinder extending longitudinally in a straight manner or according to an open broken line.

Advantageously, neck 2b may have a constant or variable inner and/or outer section.

In particular, neck 2b extends longitudinally from the second end 22 until it couples with tip 2a, which in turn extends from the coupling portion with neck 2b to the first head end 21.

Tip 2a and neck 2b are suitably sizeable (e.g. sphere radius, length or angle of the broken line) for adapting the handpiece to a plurality of applications.

Preferably, neck 2b has a length of between 100mm and 160mm.

Preferably, neck 2b has an annular section having an outer diameter of between 10mm and 14mm.

Preferably, neck 2b extends longitudinally and has a bending angle of between 20° and 30°.

According to a preferred embodiment, the electrical connection means 4 are configured to connect with an external unit 8 for radio-frequency or ultrasound treatments.

Such an external unit 8 is configured to power body 2 of handpiece 1.

Handle 6 extends between a front end 61 and a rear end 62 (see figures 3 and 6).

Handle 6 is configured to be gripped and carried by a user's hand.

The second end 22 of body 2 and the front end 61 of handle 6 are configured to provide an electromechanical coupling so as to be mechanically and electrically associable in a removable manner.

According to a preferred embodiment, handle 6 comprises electromechanical connection means 6a accommodated close to the front end 61 and configured to mechanically and electrically connect with the electrical connection means 4, so as to power the resonant actuator 3 via the electrical connection means 4.

According to one embodiment, the electromechanical connection between the electrical connection means 4 and the electromechanical connection means 6a takes place through an electromechanical connector of the type with mechanical bayonet coupling. For example, the electrical connection means 4 comprise a female "socket" connector, and the electromechanical connection means 6a comprise a male "plug" connector.

According to a preferred embodiment, handle 6 is shaped as an internally hollow body in which at least one processing unit 7 is accommodated. The processing unit 7 is in signal communication with the electromechanical connection means 6a, so as to be connectable with the resonant actuator 3 when the second end 22 of body 2 and the front end 61 of handle 6 are electrically and mechanically coupled.

Preferably, the internally hollow body of handle 6 is made of a biocompatible metal material (e.g. steel for biomedical use).

Preferably, handle 6 comprises connection means 6b placed close to the rear end 62 to set at least one processing unit 7 in signal communication with an external unit 8 configured to generate the appropriate signals to be conveyed to the dermis through handpiece 1.

To this end, the external unit 8 is configured to electrically power handpiece 1. Even more preferably, the connection means 6b comprise an electromechanical contact of the 4-pole circular connector type with BNC 2 poles.

Preferably, handle 6 comprises a serial port in signal communication with the processing unit 7 and with the external unit 8, to allow the monitoring and control of the operation of handpiece 1.

Even more preferably, handle 6 comprises a temperature sensor configured to detect the temperature inside handle 6, to provide a monitoring of the operation of handpiece 1.

According to one aspect of the present description, handpiece 1 comprises a plurality of bodies 2, each of which designed for a specific functionality.

In any case, each body 2 is configured, as described above, to removably and interchangeably associate with handle 6, which is unique, through the electromechanical connection.

With the possibility of using different bodies 2, it is possible to adapt handpiece 1 to a plurality of different applications, thus making it more usable.

According to an aspect, the processing unit 7 is configured to identify each body of the plurality of bodies 2, in the moment in which they are associated with handle 6.

According to a preferred solution, handpiece 1 transfers in a bipolar manner (thus without using negative poles or a plate) a sinusoidal waveform at high frequency, preferably comprised in a range of a few hundreds of kHz, with variable Vrms voltage, preferably comprised in a range of a few hundred Vrsm.

As regards the production steps of handpiece 1, it should be noted that the filling step with epoxy resin of body 2 is carried out after the connection steps of the resonant actuator 3, among which we may mention: a coating step of the resonant actuator 3 with electrical insulating material (e.g. polyamide), and a connection step with the electrical connection means 4 (e.g. highfrequency female plug).

As is seen, the use of the electromechanical coupling between body 2 and handle 6 allows making body 2 of handpiece 1 completely isolated from the outside.

Advantageously, body 2 has reduced dimensions which make it suitable for intraoral dental (e.g. gingival cavities) and cosmetic (e.g. reduced surface zones such as cheekbones or others) applications.

Of course, a man skilled in the art may make several changes to the variants of the handpiece described above in order to meet specific and incidental needs, all falling within the scope of protection as defined by the following claims.

## Claims

1. Handpiece (1) configured to emit electromagnetic radio-frequency or ultrasound waves, comprising:
- a body (2) extending along a predefined extension direction between a first head end (21) and a second end (22), said first head end (21) being configured to come into contact with an individual's body surface portion, said body (2) comprising a resonant actuator (3) configured to generate radio-frequency or ultrasound waves and electrical connection means (4) operatively connected with said resonant actuator (3) to electrically power it, said body (2) defining a cavity (23) extending between said first head end (21) and said second end (22), said resonant actuator (3) and said electrical connection means (4) being arranged in said cavity (23), said body (2) comprising an internally hollow tip (2a) placed at said first head end (21), said tip (2a) being configured to accommodate said resonant actuator (3), said body (2) comprising an internally hollow neck (2b) extending between said second end (22) and said first head end (21) so as to couple with said tip (2a) to define said inner cavity (23), said neck (2b) being configured to accommodate said electrical connection means (4),
- insulating means (5) arranged in said cavity (23) to make said body (2) sterilizable in autoclave, **characterized in that**
- said tip (2a) is made of a conductive material so as to form a negative pole (N) and comprises electrically insulating means (5a) placed in at least a portion of said tip (2a) so as to form an insulated positive pole (P) .

2. Handpiece (1) according to claim 1, wherein said insulating means (5) are arranged close to said first head end (21) and to said second end (22) of said body (2) or spread in said cavity (23) so as to enclose said resonant actuator (3) and said electrical connection means (4).

3. Handpiece (1) according to claim 1 or 2, wherein said insulating means (5) comprise an insulating polymer material.

4. Handpiece (1) according to claim 3, wherein said insulating polymer material comprises an insulating polymer resin.

5. Handpiece (1) according to any one of the preceding claims, wherein said resonant actuator (3) is arranged close to said first head end (21), and said electrical connection means (4) are arranged close to said second end (22).

6. Handpiece (1) according to any one of the preceding claims, comprising a handle (6) extending between a front end (61) and a rear end (62) and configured to be gripped by a user, said second end (22) of said body (2) and said front end (61) of said handle (6) are configured to form an electromechanical coupling so as to be mechanically and electrically associable in a removable manner.

7. Handpiece (1) according to claim 6, wherein said handle (6) comprises electromechanical connection means (6a) accommodated close to said front end (61) and configured to mechanically and electrically connect with said electrical connection means (4), so as to power said resonant actuator (3) via said electrical connection means (4).

8. Handpiece (1) according to claim 7, wherein said handle (6) is shaped as a hollow body and comprises at least one processing unit (7) accommodated therein, said processing unit (7) is in signal communication with said electromechanical connection means (6a), so as to be connectable with said resonant actuator (3) when said second end (22) of said body (2) and said front end (61) of said handle (6) are electrically and mechanically coupled.

9. Handpiece (1) according to claim 8, wherein said handle (6) comprises connection means (6b) placed close to said rear end (62) to set said at least one processing unit (7) in signal communication with an external unit (8) for radio-frequency or ultrasounds, said external unit (8) being configured to power said handpiece (1).

## Patentansprüche

1. Handstück (1), das konfiguriert ist, um elektromagnetische Hochfrequenz- oder Ultraschallwellen zu emittieren, umfassend:
- einen Körper (2), der sich entlang einer vordefinierten Erstreckungsrichtung zwischen einem ersten Kopfende (21) und einem zweiten Ende (22) erstreckt, wobei das erste Kopfende (21) so konfiguriert ist, dass es mit dem Körperoberflächenabschnitt einer Person in Kontakt kommt; wobei der Körper (2) einen zum Erzeugen von Radiowellen oder Ultraschallwellen ausgebildeten Resonanzaktuator (3) und mit dem Resonanzaktuator (3) operativ verbundene elektrische Verbindungsmittel (4) umfasst, wobei der Körper (2) einen Hohlraum (23) definiert, der sich zwischen dem ersten Kopfende (21) und dem zweiten Ende (22) erstreckt, wobei der Resonanzaktuator (3) und die elektrischen Verbindungsmittel (4) in dem Hohlraum (23) angeordnet sind, wobei der Körper (2) eine innen hohle Spitze (2a) definiert, die am ersten Kopfende (21) angeordnet ist, wobei die Spitze (2a) konfiguriert ist, um den Resonanzaktuator (3) aufzunehmen, wobei der Körper (2) einen sich zwischen dem zweiten Ende (22) und dem ersten Kopfende (21) erstreckenden innen hohlen Hals (2b) umfasst, um mit der Spitze (2a) gekoppelt zu werden um den inneren Hohlraum (23) zu definieren, wobei der Hals (2b) konfiguriert ist, um die elektrischen Verbindungsmittel (4) aufzunehmen,
- Isoliermittel (5), die in dem Hohlraum (23) angeordnet sind, um den Körper (2) in einem Autoklaven sterilisierbar zu machen,
**dadurch gekennzeichnet, dass**
- die Spitze (2a) aus einem leitenden Material hergestellt wird, um einen negativen Pol (N) zu bilden, und elektrisch isolierende Mittel (5a) aufweist, die in mindestens einem Teil der Spitze (2a) angeordnet sind, um einen isolierten positiven Pol (P) zu bilden.

2. Handstück (1) nach Anspruch 1, wobei die Isoliermittel (5) nahe dem ersten Kopfende (21) und dem zweiten Ende (22) des Körpers (2) angeordnet sind oder im Hohlraum (23) ausgebreitet sind, um den Resonanzaktuator (3) und die elektrischen Verbindungsmittel (4) einzuschließen.

3. Handstück (1) nach Anspruch 1 oder 2, wobei die Isoliermittel (5) ein isolierendes Polymermaterial umfassen.

4. Handstück (1) nach Anspruch 3, wobei das isolierende Polymermaterial ein isolierendes Polymerharz umfasst.

5. Handstück (1) nach einem der vorhergehenden Ansprüche, wobei der Resonanzaktuator (3) nahe dem ersten Kopfende (21) angeordnet ist und die elektrischen Verbindungsmittel (4) nahe dem zweiten Ende (22) angeordnet sind.

6. Handstück (1) nach einem der vorhergehenden Ansprüche, mit einem Handgriff (6), der sich zwischen einem vorderen Ende (61) und einem hinteren Ende (62) erstreckt und von einem Benutzer ergriffen werden kann, wobei das zweite Ende (22) des Körpers (2) und das vordere Ende (61) des Handgriffs (6) so konfiguriert sind, dass sie eine elektromechanische Kupplung bilden, so dass sie mechanisch und elektrisch entfernbar verbunden werden können.

7. Handstück (1) nach Anspruch 6, wobei der Handgriff (6) elektromechanische Verbindungsmittel (6a) umfasst, die nahe dem vorderen Ende (61) angeordnet sind und so konfiguriert sind, dass sie mechanisch und elektrisch mit den elektrischen Verbindungsmittel (4) verbunden sind, um der Resonanzaktuator (3) über die elektrische Verbindungsmittel (4) anzutreiben.

8. Handstück (1) nach Anspruch 7, **dadurch gekennzeichnet, dass** der Handgriff (6) hohlkörperförmig ausgebildet ist und mindestens eine darin aufgenommene Verarbeitungseinheit (7) umfasst, wobei die Verarbeitungseinheit (7) mit den elektromechanischen Verbindungsmitteln (6a) in Signalverbindung steht, um mit dem Resonanzaktuator (3) verbindbar zu sein, wenn das zweite Ende (22) des Körpers (2) und das vordere Ende (61) des Handgriffs (6) elektrisch und mechanisch gekoppelt sind.

9. Handstück (1) nach Anspruch 8, wobei der Handgriff (6) Verbindungsmittel (6b) aufweist, die nahe dem hinteren Ende (62) angeordnet sind, um die mindestens eine Verarbeitungseinheit (7) in Signalverbindung mit einer externen Radiofrequenz- oder Ultraschall-Einheit (8) zu setzen, wobei die externe Einheit (8) konfiguriert ist, um das Handstück (1) anzutreiben.

## Revendications

1. Pièce à main (1) configurée pour émettre des ondes électromagnétiques à radiofréquence ou à ultrasons, comprenant :
- un corps (2) qui s'étend le long d'une direction d'extension prédéfinie entre une première extrémité de tête (21) et une seconde extrémité (22), où ladite première extrémité de tête (21) est configurée pour entrer en contact avec une partie de la surface du corps (2) d'un individu, où ce corps (2) comprend un actionneur résonnant (3) configuré pour générer des ondes à radiofréquences ou à ultrasons, et des moyens de connexion électriques (4) fonctionnellement connectés audit actionneur résonnant (3) pour l'alimenter électriquement, où ledit corps (2) définit une cavité (23) s'étendant entre ladite première extrémité de tête (21) et ladite seconde extrémité (22), où ledit actionneur de résonance (3) et lesdits moyens de connexion électriques (4) sont agencés dans ladite cavité (23), où ledit corps (2) comprend une pointe interne creuse (2a) placée à ladite première extrémité de tête (21), où ladite pointe (2a) est configurée pour recevoir ledit actionneur résonnant (3), où ledit corps (2) comprend un col creux interne (2b) s'étendant entre ladite seconde extrémité (22) et ladite première extrémité de tête (21) de manière à se coupler avec ladite pointe (2a) pour définir ladite cavité interne (23), où ledit col (2b) est configuré pour recevoir lesdits moyens de connexion électriques (4),
- des moyens d'isolation (5) agencés dans ladite cavité (23) pour rendre ledit corps (2) stérilisable en autoclave,
**caractérisée en ce que**
- ladite pointe (2a) est réalisée en un matériau conducteur de manière à former un pôle négatif (N) et comprend des moyens électriquement isolants (5a) placés dans au moins une partie de ladite pointe (2a) de manière à former un pôle positif isolé (P).

2. Pièce à main (1) selon la revendication 1, dans laquelle lesdits moyens isolants (5) sont disposés à proximité de ladite première extrémité de tête (21) et de ladite seconde extrémité (22) dudit corps (2) ou s'étalent dans ladite cavité (23) de façon à enfermer ledit actionneur résonnant (3) et lesdits moyens de connexion électriques (4).

3. Pièce à main (1) selon les revendications 1 ou 2, dans laquelle lesdits moyens isolants (5) comprennent un matériau polymère isolant.

4. Pièce à main (1) selon la revendication 3, dans laquelle ledit matériau polymère isolant comprend une résine polymère isolante.

5. Pièce à main (1) selon l'une quelconque des revendications précédentes, dans laquelle ledit actionneur résonnant (3) est agencé à proximité de ladite première extrémité de tête (21) et lesdits moyens de connexion électriques (4) sont disposés à proximité de ladite seconde extrémité (22).

6. Pièce à main (1) selon l'une quelconque des revendications précédentes, comprenant une poignée (6) s'étendant entre une extrémité avant (61) et une extrémité arrière (62) et configurée pour être saisie par un utilisateur, où ladite seconde extrémité (22) dudit corps (2) et ladite extrémité avant (61) de ladite poignée (6) sont configurées pour former un couplage électromécanique de manière à pouvoir être associées mécaniquement et électriquement de manière amovible.

7. Pièce à main (1) selon la revendication 6, dans laquelle ladite poignée (6) comprend des moyens de connexion électromécaniques (6a) placés à proximité de ladite extrémité avant (61) et configurés pour se connecter mécaniquement et électriquement avec lesdits moyens de connexion électriques (4) pour alimenter ledit actionneur résonnant (3) par l'intermédiaire desdits moyens de connexion électriques (4).

8. Pièce à main (1) selon la revendication 7, dans laquelle ladite poignée (6) est conformée en corps creux et comprend au moins une unité de traitement (7) logée dans celui-ci, ladite unité de traitement (7) est en communication de signal avec lesdits moyens de connexion électromécaniques (6a) de manière à être connectable avec ledit actionneur résonnant (3) lorsque ladite seconde extrémité (22) dudit corps (2) et ladite extrémité avant (61) de ladite poignée (6) sont couplées électriquement et mécaniquement.

9. Pièce à main (1) selon la revendication 8, dans laquelle ladite poignée (6) comprend des moyens de connexion (6b) placés à proximité de ladite extrémité arrière (62) pour mettre en communication ladite au moins une unité de traitement (7) avec une unité externe (8) pour des radiofréquences ou des ultrasons, ladite unité externe (8) étant configurée pour alimenter ladite pièce à main (1).
